# EUROPEAN PATENT SPECIFICATION

(11) **EP 2 830 713 B1**
(45) Date of publication and mention of the grant of the patent: **25.10.2017**
(21) Application number: 13715859.8
(22) Date of filing: 28.03.2013
(51) Int. Cl.: A61Q 5/12, A61K 8/90, A61K 8/41, A61K 8/42, A61K 8/34, A61K 8/81, A61K 8/891, A61K 8/898

(54) **HAIR CONDITIONING COMPOSITION COMPRISING MONO-ALKYL AMINE CATIONIC SURFACTANT SYSTEM, DEPOSITION POLYMER, AND SILICONE**
HAARKONDITIONIERUNGSZUSAMMENSETZUNGEN MIT EINEM KATIONISCHEN MONOALKYL-AMIN TENSID SYSTEM, EINEM ABSCHEIDUNGSPOLYMER UND SILICON
PRÉPARATION DE CONDITIONNEMENT DES CHEVEUX AVEC UN SYSTÈME TENSIOACTIF CATIONIQUE DE TYPE MONOALKYL-AMIN, POLYMERE DE DÊPOT ET SILICONE

(30) Priority: 30.03.2012 US 201261617669 P
(43) Date of publication of application: 04.02.2015
(73) Proprietor: The Procter & Gamble Company, Cincinnati, OH 45202 (US)
(72) Inventor: UEHARA, Nobuaki, Singapore 138648 (SG); KRISHAN, Kapilanjan, Kobe Hyogo 658-0032 (JP)
(74) Representative: Sauvaître, Thibault Bruno
(86) International application number: PCT/US2013/034198
(87) International publication number: WO 2013/148904

(56) References cited:
- EP-A2- 0 668 070
- WO-A1-2004/030646
- WO-A2-99/34768
- WO-A2-2009/016555
- US-A1- 2006 210 509
- US-A1- 2008 286 218

## Description

### FIELD OF THE INVENTION

The present invention relates to a hair conditioning composition according to the claims as appended comprising: a mono-alkyl amine cationic surfactant; a di-alkyl quaternized ammonium salt cationic surfactant; a high melting point fatty compound; a deposition polymer having specific monomers; a silicone compound; and an aqueous carrier. The composition of the present invention provides improved deposition of cationic surfactant, fatty compounds, and/or silicone compounds, especially silicone compounds on damaged hair.

### BACKGROUND OF THE INVENTION

A variety of approaches have been developed to condition the hair. See e.g. US2006/210509, US2008/286218, WO2009/016555, WO2004/030646, EP0668070, WO99/34768. A common method of providing conditioning benefit is through the use of conditioning agents such as cationic surfactants, high melting point fatty compounds, silicone compounds, and mixtures thereof. Most of these conditioning agents are known to provide various conditioning benefits.

There have been trials for conditioners to provide improved conditioning benefits. For example, Japanese Patent Application Laid-Open No. 2007-137830 discloses hair cosmetics comprising a cationic surfactant, a fatty alcohol, a silicone, and a polymer containing hydrophilic nonionic monomers and anionic monomers. Japanese Patent Application Laid-Open No. 2007-137830 also describes that such hair cosmetics provide superior conditioning benefits.

However, there is still a need for rinse-off conditioners to provide improved deposition of conditioning agents on the hair, especially on damaged hair. By improved deposition of conditioning agents, such rinse-off conditioners can provide either: improved conditioning benefits from the same amount of the conditioning agents; or conditioning benefits effectively from the reduced amount of the conditioning agents.

None of the existing art provides all of the advantages and benefits of the present invention.

### SUMMARY OF THE INVENTION

The present invention is directed to a hair conditioning composition comprising by weight:
(a) from about 0.1% to about 8% of a primary, secondary or tertiary alkyl amine cationic surfactant having one long alkyl or alkenyl group of from about 12 to about 30 carbon atoms;
(b) from about 0.05% to about 6% of a di-alkyl quaternized ammonium salt cationic surfactant said di-alkyl groups being two long alkyl groups of from about 12 to about 30 carbon atoms;
(c) from about 1% to about 15% of a high melting point fatty compound;
(d) from about 0.05% to about 6% of a deposition polymer which is a copolymer comprising: a vinyl monomer (A) with a carboxyl group in the structure; and a vinyl monomer (B) expressed by the following formula (1):

   CH₂=C(R¹)-CO-X-(Q-O)ᵣ-R² (1)

   wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and wherein the vinyl monomer (A) is contained at a level of from about 10 mass% to about 50 mass%, and the vinyl monomer (B) is contained at level of from about 50 mass% to about 90 mass%; and
(e) from about 0.05% to about 15% of a silicone compound; and
(f) an aqueous carrier.

The composition of the present invention provides improved deposition of cationic surfactants, fatty compounds, and/or silicone compounds, especially silicone compounds on damaged hair. Damaged hairs herein include, for example, hair tips rather than hair root, chemically damaged hair by bleaching, coloring and/or perming, and/or physically damaged by combing.

The inventors of the present invention have found that, by the use of the two specific cationic surfactants, together with the specific deposition polymer, the composition of the present invention provides improved deposition, compared to other compositions such as those containing different cationic surfactants and/or different deposition polymers.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### MONO-ALKYL AMINE CATIONIC SURFACTANT

The mono-alkyl amine cationic surfactant is included in the composition at a level of from about 0.1% to about 8%, preferably from about 0.2% to about 6%, more preferably from about 0.5% to about 5% by weight of the composition.

Mono-alkyl amine cationic surfactants useful herein are primary, secondary, and tertiary amines having one long alkyl or alkenyl group of from about 12 to about 30 carbon atoms, preferably from 16 to 24 carbon atoms, more preferably from 18 to 22 alkyl group. Mono-alkyl amines useful herein also include mono-alkyl amidoamines.
Particularly useful are tertiary amidoamines having an alkyl group of from about 12 to about 22 carbon atoms, preferably from about 16 to about 22 carbon atoms. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al.

The above mono-alkyl amine cationic surfactants are preferably used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The acid can be used at a molar ratio of the amine to the acid of from about 1 : 0.3 to about 1 : 2, more preferably from about 1 : 0.4 to about 1 : 1.

### DI-ALKYL QUATERNIZED AMMONIUM SALT CATIONIC SURFACTANT

The di-alkyl quaternized ammonium salt cationic surfactant is included in the composition at a level of from about 0.05% to about 5%, preferably from about 0.1% to about 4%, more preferably from about 0.2% to about 3% by weight of the composition. It is preferred that the weight ratio of the mono-alkyl amine cationic surfactant to the di-alkyl quatenized ammonium salt cationic surfactant is from about 1:1 to about 5:1, more preferably from about 1.2:1 to about 5:1, still more preferably from about 1.5:1 to about 4:1, in view of stability in rheology and conditioning benefits.

Di-alkyl quaternized ammonium salt cationic surfactants useful herein are those having two long alkyl chains of from 12 to 30 carbon atoms, more preferably from 16 to 24 carbon atoms, still more preferably from 18 to 22 carbon atoms. Such di-alkyl quaternized ammonium salts useful herein are those having the formula (I): 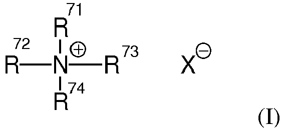 wherein two of R⁷¹, R⁷², R⁷³ and R⁷⁴ are selected from an aliphatic group of from 12 to 30 carbon atoms, preferably from 16 to 24 carbon atoms, more preferably from 18 to 22 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, preferably from 1 to 3 carbon atoms or an aromatic, alkoxy, polyoxyalkylene, alkylamido, hydroxyalkyl, aryl or alkylaryl group having up to about 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The composition of the present invention comprises a high melting point fatty compound. The high melting point fatty compound is included in the composition at a level of from about 1% to about 15%, preferably from about 1.5% to about 12%, more preferably from about 2% to about 10% by weight of the composition.

The high melting point fatty compound useful herein have a melting point of 25°C or higher, preferably 40°C or higher, more preferably 45°C or higher, still more preferably 50°C or higher, in view of stability of the emulsion especially the gel matrix. Preferably, such melting point is up to about 90°C, more preferably up to about 80°C, still more preferably up to about 70°C, even more preferably up to about 65°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols.

Preferred fatty alcohols include, for example, cetyl alcohol (having a melting point of about 56°C), stearyl alcohol (having a melting point of about 58-59°C), behenyl alcohol (having a melting point of about 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group. In the present invention, more preferred fatty alcohols are cetyl alcohol, stearyl alcohol and mixtures thereof.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan).

### GEL MATRIX

Preferably, in the present invention, a gel matrix is formed by the cationic surfactants, the high melting point fatty compound, and an aqueous carrier. The gel matrix is suitable for providing various conditioning benefits, such as slippery feel during the application to wet hair and softness and moisturized feel on dry hair.

Preferably, especially when the gel matrix is formed, the total amount of the cationic surfactant and the high melting point fatty compound is from about 4.5%, preferably from about 5.0%, more preferably from about 5.5% by weight of the composition, in view of providing the benefits of the present invention, and to about 15%, preferably to about 14%, more preferably to about 13%, still more preferably to about 10% by weight of the composition, in view of spreadability and product appearance. Furthermore, when the gel matrix is formed, the cationic surfactant and the high melting point fatty compound are contained at a level such that the weight ratio of the cationic surfactant to the high melting point fatty compound is in the range of, preferably from about 1:1 to about 1:10, more preferably from about 1:1.5 to about 1:7, still more preferably from about 1:2 to about 1:6, in view of providing improved wet conditioning benefits.

Preferably, when the gel matrix is formed, the composition of the present invention is substantially free of anionic surfactants, in view of stability of the gel matrix. In the present invention, "the composition being substantially free of anionic surfactants" means that: the composition is free of anionic surfactants; or, if the composition contains anionic surfactants, the level of such anionic surfactants is very low. In the present invention, a total level of such anionic surfactants, if included, preferably 1% or less, more preferably 0.5% or less, still more preferably 0.1% or less by weight of the composition. Most preferably, the total level of such anionic surfactants is 0% by weight of the composition.

### AQUEOUS CARRIER

The composition of the present invention comprises an aqueous carrier. The level and species of the carrier are selected according to the compatibility with other components, and other desired characteristic of the product.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 99%, preferably from about 30% to about 95%, and more preferably from about 80% to about 90% water.

### DEPOSITION POLYMER

The composition of the present invention further comprises a deposition polymer, preferable anionic deposition polymer. The deposition polymer is included at a level by weight of the composition of, from about 0.05% to about 8%, preferably from about 0.1% to about 5%, more preferably from about 0.2% to about 3.5%.

It is preferred that the weight ratio of (i) the deposition polymer to (ii) a sum of the mono-alkyl amine salt cationic surfactant, di-alkyl quaternized ammonium salt cationic surfactant, and high melting point fatty compound is from about 1:1 to about 1:160, more preferably from about 1:2.5 to about 1:120, still more preferably from about 1:3.5 to about 1:80. If the weight ratio of (i) to (ii) is too low, the composition may provide lower deposition of cationic surfactants, high melting point fatty compounds, and/or silicone compounds. If the weight ratio of (i) to (ii) is too high, the composition may influence rheology, and may undesirably decrease rheology of the composition.

The deposition polymer useful herein is a copolymer comprising: a vinyl monomer (A) with a carboxyl group in the structure; and a vinyl monomer (B) expressed by the following formula (1):

CH₂=C(R¹)-CO-X-(Q-O)ᵣ-R² (1)

wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and
wherein the vinyl monomer (A) is contained at a level of from about 10 mass% to about 50 mass%, and the vinyl monomer (B) is contained at level of from about 50 mass% to about 90 mass%.

### Vinyl Monomer (A)

The copolymer of the present invention contains a vinyl monomer (A) having a carboxyl group in the structure. The copolymer may contain one type of the vinyl monomer (A), or may contain two or more types of the vinyl monomer (A). The vinyl monomer (A) is preferably anionic.

This vinyl monomer (A) is contained at a level of from about 10 mass% based on the total mass of the copolymer, preferably from about 15 mass%, more preferably 20 mass% or higher, and even more preferably 25 mass% or higher, in view of improved deposition of cationic surfactants, fatty compounds and/or silicones, and to about 50 mass%, preferably 45 mass% or less, and more preferably 40 mass% or less, in view of not-deteriorating smoothness during application and/or the product viscosity.

Non-limited example of the vinyl monomer (A) having a carboxyl group include, for example, unsaturated carboxylic acid monomers having 3 to 22 carbon atoms. The unsaturated carboxylic acid monomer has, preferably 4 or more carbon atoms, and preferably 20 or less carbon atoms, more preferably 18 or less carbon atoms, still more preferably 10 or less carbon atoms, and even more preferably 6 or less carbon atoms. Furthermore, the number of carboxyl groups in the vinyl monomer (A) is preferably from 1 to 4, more preferably from 1 to 3, even more preferably from 1 to 2, and most preferably 1.

In view of improved deposition of cationic surfactants, fatty compounds and/or silicones, the vinyl monomer (A) is preferably an unsaturated carboxylic acid monomer expressed by the following formula (2) or formula (3), more preferably those expressed by the formula (2).

CH₂=C(R³)-CO-(O-(CH₂)ₘ-CO)ₙ-OH (2)

wherein: R³ represents a hydrogen atom or a methyl group, preferably a hydrogen atom; m represents an integer of 1 through 4, preferably 2 to 3; and n represents an integer of 0 through 4, preferably 0 to 2, and most preferably 0.

CH₂=C(R⁴)-COO-(CH₂)ₚ-OOC-(CH₂)_{q}-COOH (3)

wherein: R⁴ represents a hydrogen atom or a methyl group, preferably a hydrogen atom; p and q independently represent an integer of 2 through 6, preferably 2 to 3.

Examples of those expressed by the formula (2) include (meth)acrylic acid, crotonic acid, maleic acid, fumaric acid, itaconic acid, angelic acid, tiglic acid, 2-carboxy ethyl acrylate oligomer, and the like. Among them, preferred are acrylic acid and methacrylic acid, and more preferred is acrylic acid. Examples of those expressed by the formula (3) include acryloyloxy ethyl succinate, 2-methacryloyloxy ethyl succinate, and the like.

### Vinyl Monomer (B)

The copolymer contains a vinyl monomer (B). The copolymer may contain one type of the vinyl monomer (B), or may contain two or more types of the vinyl monomer (B). The vinyl monomer (B) is preferably nonionic.

The vinyl monomer (B) is contained at a level of from about 50 mass% based on the total mass of the copolymer in view of improving the feel and the smoothness during application, and to about 90 mass% based on the total mass of the copolymer, preferably to about 85 mass%, more preferably to about 80 mass%, still more preferably 75 mass%, in view of improved deposition of cationic surfactants, fatty compounds and/or silicones.

The Vinyl monomers (B) useful herein are those expressed by formula (4).

CH₂=C(R¹)-CO-X-(Q-O)ᵣ-R² (4)

wherein: R¹ represents a hydrogen atom or a methyl group; R² represents a hydrogen atom or an alkyl group with 1 through 5 carbon atoms, which may have a substitution group; Q represents an alkylene group with 2 through 4 carbon atoms which may also have a substitution group; r represents an integer from 2 through 15; and X represents an oxygen atom or an NH group; and in the structure - (Q - O)ᵣ - R², the number of atoms bonded in a straight chain is 70 or less.

If R² has a substitution group, the substitution group is a substitution group that does not react with other parts of the copolymer. The vinyl monomer (B) is preferably hydrophilic, and therefore R² is preferably a hydrogen atom or an alkyl group with 1 ∼ 3 carbon atoms, and more preferably a hydrogen atom or an alkyl group with 1 or 2 carbon atoms.

X preferably represents an oxygen atom.

Q represents preferably an alkylene group with 2 through 3 carbon atoms which may also have a substitution group, and more preferably an alkylene group with 2 through 3 carbon atoms without any substitution group. If the alkylene group of Q has a substitution group, it is preferred that such substitution group does not react with other parts of the copolymer, more preferably such substitution group has a molecular weight of 50 or less, still more preferably such substitution group has a molecular weight that is smaller than the structural moiety of - (Q - O)ᵣ -. Examples of such substitution group include a hydroxyl group, methoxy group, ethoxy group, and the like.

r represents preferably 3 or higher, and preferably 12 or less, in view of improved deposition of cationic surfactants, fatty compounds and/or silicones, and/or in view of smoothness during application.

As described above, in the structure - (Q - O)ᵣ - R², the number of atoms that are bonded by the straight chain is 70 or less. For example, if Q represents an n-butylene group, r = 15, and R² represents an n-pentyl group, the number of atoms that are bonded in the straight chain of the structure - (Q - O)ᵣ - R² is calculated as 80, which therefore is outside of the scope. The number of atoms bonded in the straight chain in the structure - (Q - O)ᵣ - R² is preferably 60 or less, more preferably 40 or less, even more preferably 28 or less, and particularly preferably 20 or less, in view of improved deposition of cationic surfactants, fatty compounds and/or silicones, and/or in view of smoothness during application.

Examples of the vinyl monomer (B) include, methoxy polyethylene glycol (meth)acrylate (where the number of repetitions of polyethylene glycol (r in formula (4)) is between 2 ∼ 15), polyethylene glycol (meth)acrylate (where the number of repetitions of polyethylene glycol (r in formula (4)) is between 2 ∼ 15), methoxy polyethylene glycol / polypropylene glycol (meth)acrylate (where the number of repetitions of polyethylene glycol / polypropylene glycol (r in formula (4)) is between 2 ∼ 15), polyethylene glycol / polypropylene glycol (meth)acrylate (where the number of repetitions of polyethylene glycol / polypropylene glycol (r in formula (4)) is between 2 ∼ 15), methoxy polyethylene glycol / polybutylene glycol (meth)acrylate (where the number of repetitions of polyethylene glycol / polybutylene glycol (r in formula (4)) is between 2 ∼ 15), polyethylene glycol / polybutylene glycol (meth)acrylate (where the number of repetitions of polyethylene glycol / polybutylene glycol (r in formula (4)) is between 2 ∼ 15), methoxy polyethylene glycol (meth)acrylamide (where the number of repetitions of polyethylene glycol (r in formula (4)) is between 2 ∼ 15), and polyethylene glycol (meth)acrylamide (where the number of repetitions of polyethylene glycol (r in formula (4)) is between 2 ∼ 15); preferably methoxy polyethylene glycol (meth)acrylate (where the number of repetitions of polyethylene glycol (r in formula (4)) is between 3 ∼ 12), polyethylene glycol (meth)acrylate (where the number of repetitions of polyethylene glycol (r in formula (4)) is between 3 ∼ 12), methoxy polyethylene glycol / polypropylene glycol (meth)acrylate (where the number of repetitions of polyethylene glycol / polypropylene glycol (r in formula (4)) is between 3 ∼ 12), polyethylene glycol / polypropylene glycol (meth)acrylate (where the number of repetitions of polyethylene glycol / polypropylene glycol (r in formula (4)) is between 3 ∼ 12), methoxy polyethylene glycol / polybutylene glycol (meth)acrylate (where the number of repetitions of polyethylene glycol / polybutylene glycol (r in formula (4)) is between 3 ∼ 12), polyethylene glycol / polybutylene glycol (meth)acrylate (where the number of repetitions of polyethylene glycol / polybutylene glycol (r in formula (4)) is between 3 ∼ 12); more preferably methoxy polyethylene glycol (meth)acrylate (where the number of repetitions of polyethylene glycol (r in formula (4)) is between 3 ∼ 12), and polyethylene glycol (meth)acrylate (where the number of repetitions of polyethylene glycol (r in formula (4)) is between 3 ∼ 12).

### Vinyl Monomer (C)

In addition to the vinyl monomers (A) and (B), the copolymer may further contain a vinyl monomer (C) having an alkyl group with 12 ∼ 22 carbon atoms, in view of providing conditioning effect such as smoothness during application. When included, the amount of the vinyl monomer (C) is preferably 40 mass% or less, more preferably 30 mass% or less, even more preferably 25 mass% or less, and still more preferably 20 mass% or less based on the total mass of the copolymer, in view of improved deposition of cationic surfactants, fatty compounds and/or silicones, and/or in view of smoothness during application.

Preferably, the vinyl monomer (C) is a (meth)acrylate monomer having an alkyl group with 12 ∼ 22 carbon atoms, in view of smoothness during application. Furthermore, vinyl monomers with branched alkyl groups are particularly preferred.

Examples of the (meth)acrylate monomer having an alkyl group with 12 ∼ 22 carbon atoms include myristyl (meth)acrylate, isostearyl (meth)acrylate, stearyl (meth)acrylate, behenyl (meth)acrylate, cetyl (meth)acrylate, lauryl (meth)acrylate, synthetic lauryl (meth)acrylate, (however "synthetic lauryl (meth)acrylate" refers to an alkyl (meth)acrylate having alkyl groups with 12 carbon atoms and alkyl groups with 13 carbon atoms), and the like. Of these, (meth)acrylate monomers having an alkyl group with 12 ∼ 20 carbon atoms are preferable, and (meth)acrylate monomers having an alkyl group with 16 ∼ 18 carbon atoms are more preferable.

The copolymer may contain one type of the vinyl monomer (C), or may contain two or more types of the vinyl monomer (C).

### Other Monomers

In addition to the aforementioned vinyl monomers (A), (B), and (C), the copolymer may also contain other vinyl monomers, to the extent not to deteriorate the effect of the copolymer. Examples of other vinyl monomers include nonionic monomers, amphoteric monomers, semipolar monomers, cationic monomers, as well as monomers containing a polysiloxane group., preferably nonionic monomers with or without polysiloxane group These other monomers are different from any of the aforementioned vinyl monomers (A), (B), and (C).

Normally the amount of such other monomers, if included, is 40 mass% or less of the total mass of the copolymer, preferably 30 mass% or less, more preferably 20 mass% or less, and even more preferably 10 mass% or less.

In view of improved deposition of cationic surfactants, fatty compounds, and/or silicones, the amount of cationic functional groups in the copolymer is preferably low, and for example cationic functional groups preferably account for 10 mole % or less of all functional groups in the copolymer. More preferably, the copolymer is free of cationic functional groups.

Examples of nonionic monomers include esters of (meth)acrylic acid and alcohols with 1 ∼ 22 carbon atoms, amides of (meth)acrylic acid and alkyl amines with 1 ∼ 22 carbon atoms, monoesters of (meth)acrylic acid and ethylene glycol, 1,3-propylene glycol or the like, as well as esters where the hydroxyl group of the monoester has been etherified by methanol, ethanol or the like, (meth)acryloyl morpholine and the like.

Examples of amphoteric monomers include (meth)acryl esters having a betaine group, (meth)acrylamide having a betaine group and the like.

Examples of semipolar monomers include (meth)acrylate esters having an amine oxide group, (meth)acrylamides having an amine oxide group, and the like.

Examples of cationic monomers include (meth)acrylate esters having a quaternary ammonium group, (meth)acrylamides having a quaternary ammonium group and the like.

The monomer containing a polysiloxane group is a monomer having a polysiloxane structure and also having a structure that can bond by covalent bond to the copolymer. These component units have high affinity towards silicone oil that is normally used in conjunction in cosmetic material compositions, and are thought to act by bonding the silicone oil to the other component units in the copolymer and thus increasing the adsorption force of silicone oil to the skin and hair, particularly damaged hair.

The polysiloxane structure is a structure where two or more repeating structural units expressed by the following formula (4) are linked.

-(SiR⁵R⁶-O)- (4)

In formula (4), R⁵ and R⁶ independently represent an alkyl group with 1 to 3 carbon atoms or a phenyl group.

The structure that can link via covalent bond to the copolymer can be a structure that has a vinyl structure such as a (meth)acrylate ester, or (meth)acrylamide and that can copolymerize with another monomer, a structure that has a functional group such as a thiol, that can link to the copolymer by chain transfer during polymerization, or a structure that has an isocyanate group, carboxylic acid group, hydroxyl group, amino group, or the like, and that can react and link to the functional groups on the copolymer, but there is no restriction to these structures.

A plurality of these linkable structures can be present in one monomer containing a polysiloxane group. In the copolymer, the polysiloxane structure can link by a graft structure to the main chain, or conversely the polysiloxane structure can be the main chain with the other structure link by a graft structure, and in addition the polysiloxane structure and the other structure can be linked in a straight chain condition by a block structure.

The monomer containing a polysiloxane group is preferably expressed by the following formula (5).

CH₂=C(R⁷)-Z-(SiR⁸R⁹-O)ₛ-R¹⁰ (5)

In the formula, R⁷ represents a hydrogen atom or a methyl group, R⁸ and R⁹ independently represent an alkyl group with 1 to 3 carbon atoms or a phenyl group, R¹⁰ represents an alkyl group with 1 to 8 carbon atoms, Z represents a bivalent linking group or a direct bond, and s represents an integer between 2 to 200.

More preferably, s is 3 or higher, and even more preferably, s is 5 or higher, in view of increased affinity to silicone oil, and preferably s is 50 or less, in view of enhanced copolymerization with the other monomers.

Z represents a bivalent linking group or a direct bond, but a linking group containing one or a combination of two or more of the structures suggested below is preferable. The numbers that are combined is not particularly restricted, but normally is 5 or less. Furthermore, the direction of the following structures are arbitrary (the polysiloxane group side can be on either end). Note, in the following, R represents an alkylene group with 1 to 6 carbon atoms or a phenylene group.

-COO-R-

-CONH-R-

-O-R-

-R-

The monomer expressed by the aforementioned formula (5), include, for example, α-(vinyl phenyl) polydimethyl siloxane, α-(vinyl benzyloxy propyl) polydimethyl siloxane, α-(vinyl benzyl) polymethyl phenyl siloxane, α-(methacryloyl oxypropyl) polydimethyl siloxane, α-(methacryloyloxy propyl) polymethyl phenyl siloxane, α-(methacryloyl amino propyl) polydimethyl siloxane and the like. The monomer containing a polysiloxane group can be a single type, or can be two or more types used in combination.

In order to adjust the molecular weight and the viscosity of the copolymer, a cross-linking agent such as a polyfunctional acrylate or the like can be introduced to the copolymer. However, in this invention, it is preferred that a cross-linking agent is not included in the copolymer.

### Structure Analysis

The amount of the vinyl monomers (A), (B), and (C) as well as other monomers in the copolymer can be measured using IR absorption or Raman scattering by the carbonyl groups, amide bonds, polysiloxane structures, various types of functional groups, carbon backbone and the like, by ¹H-NMR of methyl groups in the polydimethyl siloxane, amide bond sites, and methyl groups and methylene groups adjacent thereto, as well as various types of NMR represented by ¹³C-NMR and the like.

### Weighted Average Molecular Weight

The weighted average molecular weight of the copolymer is preferably 3,000 or higher, more preferably 5,000 or higher, and even more preferably 10,000 or higher, in view of providing conditioning effect via foaming a complex with cationic surfactant, and preferably to about 2,000,000, more preferably 1,000,000 or less, still more preferably 500,000 or less, even more preferably 100,000 or less, and most preferably 50,000 or less, in view of feeling after drying.

The weighted average molecular weight of the copolymer can be measured by gel permeation chromatography (GPC). The development solvent that is used in gel permeation chromatography is not particularly restricted so long as being a normally used solvent, but for example, the measurement can be performed using a solvent blend of water / methanol / acetic acid / sodium acetate.

### Viscosity

The copolymer preferably has a viscosity for a 20 mass% ethanol solution at 25°C of 5 mPa•s or higher and 20,000 mPa•s or less. The viscosity is more preferably 10 mPa•s or higher, even more preferably 15 mPa•s or higher, but on the other hand is more preferably 10,000 mPa•s or less, and even more preferably 5,000 mPa•s or less. The viscosity of the copolymer is preferably 5 mPa•s or higher and 20,000 mPa•s or less, from the perspective of handling. The viscosity can be measured using a B-type viscometer.

Similar to the weighted average molecular weight, the viscosity of the copolymer can be adjusted by controlling the degree of polymerization of the copolymer, and can be controlled by increasing or decreasing the amount of a cross-linking agent such as a polyfunctional acrylate or the like that is added.

### SILICONE COMPOUND

The compositions of the present invention comprise a silicone compound. The silicone compounds are included at levels by weight of the composition of from about 0.05% to about 15%, preferably from about 0.1% to about 10%, more preferably from about 0.1% to about 8%.

Preferably, the silicone compounds have an average particle size of from about 1 microns to about 50 microns, in the composition.

The silicone compounds useful herein, as a single compound, as a blend or mixture of at least two silicone compounds, or as a blend or mixture of at least one silicone compound and at least one solvent, have a viscosity of preferably from about 1,000 to about 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones including quaternized silicones comprising terminal ester groups, having a viscosity of lower than 100,000 mPa·s and a D block length of greater than 200 D units, and mixtures thereof. Other nonvolatile silicone compounds having conditioning properties can also be used.

Silicone compounds useful herein also include amino substituted materials. Preferred aminosilicones include, for example, those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R¹ is a monovalent radical conforming to the general formula C_{q}H_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻ ; wherein R₂ is hydrogen, phenyl, benzyl, or a saturated hydrocarbon radical, preferably an alkyl radical from about C₁ to about C₂₀; A is a halide ion.

Highly preferred amino silicones are those corresponding to formula (I) wherein m=0, a=1, q=3, G=methyl, n is preferably from about 1500 to about 1700, more preferably about 1600; and L is -N(CH₃)₂ or -NH₂, more preferably -NH₂. Another highly preferred amino silicones are those corresponding to formula (I) wherein m=0, a=1, q=3, G=methyl, n is preferably from about 400 to about 600, more preferably about 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to about 20,000 centistokes, preferably from about 20 to about 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from about 1,000mPa·s to about 100,000mPa·s, more preferably from about 5,000mPa·s to about 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

Silicone compounds useful herein also include polyalkyl siloxanes such as polydimethylsiloxane, polydiethylsiloxane, and polymethylphenylsiloxane. Polydimethylsiloxane, which is also known as dimethicone, is especially preferred. These silicone compounds are available, for example, from the General Electric Company in their Viscasil® and TSF 451 series, and from Dow Corning in their Dow Corning SH200 series.

The above polyalkylsiloxanes are available, for example, as a mixture with silicone compounds having a lower viscosity. Such mixtures have a viscosity of preferably from about 1,000mPa·s to about 100,000mPa·s, more preferably from about 5,000mPa·s to about 50,000mPa·s. Such mixtures preferably comprise: (i) a first silicone having a viscosity of from about 100,000mPa·s to about 30,000,000mPa·s at 25°C, preferably from about 100,000mPa·s to about 20,000,000mPa·s; and (ii) a second silicone having a viscosity of from about 5mPa·s to about 10,000mPa·s at 25°C, preferably from about 5mPa·s to about 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,000mPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

### Silicone Polymer Containing Quaternary Groups

Silicone compounds useful herein include, for example, a Silicone Polymer Containing Quaternary Groups comprising terminal ester groups, having a viscosity up to 100,000 mPa·s and a D block length of greater than 200 D units. Without being bound by theory, this low viscosity silicone polymer provides improved conditioning benefits such as smooth feel, reduced friction, and prevention of hair damage, while eliminating the need for a silicone blend.

Structurally, the silicone polymer is a polyorganosiloxane compound comprising one or more quaternary ammonium groups, at least one silicone block comprising greater than 200 siloxane units, at least one polyalkylene oxide structural unit, and at least one terminal ester group. In one or more embodiments, the silicone block may comprise between 300 to 500 siloxane units.

The silicone polymer is present in an amount of from about 0.05% to about 15%, preferably from about 0.1% to about 10%, more preferably from about 0.15% to about 5%, and even more preferably from about 0.2% to about 4% by weight of the composition.

In a preferred embodiment, the polyorganosiloxane compounds have the general formulas (Ia) and (Ib):

M-Y-[-(N⁺R₂-T-N⁺R₂)-Y-]ₘ-[-(NR²-A-E-A'-NR²)-Y-]ₖ-M (Ia)

M-Y-L-(N⁺R₂-T-N⁺R₂)-Y-]ₘ-[-(N⁺R²₂-A-E-A'-N⁺R²₂)-Y-]ₖ-M (Ib)

wherein:
m is > 0, preferred 0.01 to 100, more preferred 0.1 to 100, even more preferred 1 to 100, specifically 1 to 50, more specifically 1 to 20, even more specifically 1 to 10,
k is 0 or an average value of from >0 to 50, or preferably from 1 to 20, or even more preferably from 1 to 10,
M represents a terminal group, comprising terminal ester groups selected from

   -OC(O)-Z

   -OS(O)₂-Z

   -OS(O₂)O-Z

   -OP(O)(O-Z)OH

   -OP(O)(O-Z)₂
wherein Z is selected from monovalent organic residues having up to 40 carbon atoms, optionally comprising one or more hetero atoms.
A and A' each are independently from each other selected from a single bond or a divalent organic group having up to 10 carbon atoms and one or more hetero atoms, and
E is a polyalkylene oxide group of the general formula:

   -[CH₂CH₂O]_{q}-[CH₂CH(CH₃)O]ᵣ-[CH₂CH(C₂H₅)O]ₛ-
wherein q=0 to 200, r=0 to 200, s=0 to 200, and q+r+s = 1 to 600.
R² is selected from hydrogen or R,
R is selected from monovalent organic groups having up to 22 carbon atoms and optionally one or more heteroatoms, and wherein the free valencies at the nitrogen atoms are bound to carbon atoms,
Y is a group of the formula:

   -K-S-K- and -A-E-A'-or-A'-E-A-, 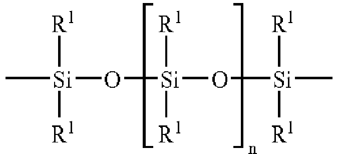
with S=
wherein R¹ = C₁-C₂₂-alkyl, C₁-C₂₂-fluoralkyl or aryl; n=200 to 1000, and these can be identical or different if several S Groups are present in the polyorganosiloxane compound.
K is a bivalent or trivalent straight chain, cyclic and/or branched C₂-C₄₀ hydrocarbon residue which is optionally interrupted by-O-,-NH-, trivalent N, -NR¹-,-C(O)-, -C(S)-, and optionally substituted with-OH, wherein R¹ is defined as above,
T is selected from a divalent organic group having up to 20 carbon atoms and one or more hetero atoms.

The residues K may be identical or different from each other. In the -K-S-K-moiety, the residue K is bound to the silicon atom of the residue S via a C-Si-bond.

Due to the possible presence of amine groups (-(NR²-A-E-A'-NR²)-) in the polyorganosiloxane compounds, they may have protonated ammonium groups, resulting from the protonation of such amine groups with organic or inorganic acids. Such compounds are sometimes referred to as acid addition salts of the polyorganosiloxane compounds.

In a preferred embodiment the molar ratio of the quaternary ammonium groups b) and the terminal ester groups c) is less than 100 : 20, even more preferred is less than 100 : 30 and is most preferred less than 100 : 50. The ratio can be determined by ¹³C-NMR.

In a further embodiment, the polyorganosiloxane composition may comprise:
A) at least one polyorganosiloxane compound, comprising a) at least one polyorganosiloxane group, b) at least one quaternary ammonium group, c) at least one terminal ester group, and d) at least one polyalkylene oxide group (as defined before),
B) at least one polyorganosiloxane compound, comprising at least one terminal ester group, different from compound A).

In the definition of component A) it can be referred to the description of the polyorganosiloxane compounds of the invention. The polyorganosiloxane compound B) differs from the polyorganosiloxane compound A) preferably in that it does not comprise quaternary ammonium groups. Preferred polyorganosiloxane compounds B) result from the reaction of monofunctional organic acids, in particular carboxylic acids, and polyorganosiloxane containing bisepoxides.

In the polyorganosiloxane compositions the weight ratio of compound A) to compound B) is preferably less than 90 : 10. Or in other words, the content of component B) is at least 10 weight percent. In a further preferred embodiment of the polyorganosiloxane compositions in compound A) the molar ratio of the quaternary ammonium groups b) and the terminal ester groups c) is less than 100 : 10, even more preferred is less than 100 : 15 and is most preferred less than 100 : 20.

The silicone polymer has a viscosity at 20°C and a shear rate of 0.1s⁻¹ (plate-plate system, plate diameter 40mm, gap width 0.5mm) of less than 100,000 mPa•s (100 Pa•s). In further embodiments, the viscosities of the neat silicone polymers may range from 500 to 100,000 mPa•s, or preferably from 500 to 70,000 mPa•s, or more preferably from 500 to 50,000 mPa•s, or even more preferably from 500 to 20,000 mPa•s. In further embodiments, the viscosities of the neat polymers may range from 500 to 10,000 mPa•s, or preferably 500 to 5000 mPa•s determined at 20°C and a shear rate of 0.1 s⁻¹.

In addition to the above listed silicone polymers, the following preferred compositions are provided below. For example, in the polyalkylene oxide group E of the general formula:

-[CH₂CH₂O]_{q}-[CH₂CH(CH₃)O]ᵣ-[CH₂CH(C₂H₅)O]ₛ-

wherein the q, r, and s indices may be defined as follows:
q=0 to 200, or preferably from 0 to 100, or more preferably from 0 to 50, or even more preferably from 0 to 20,
r=0 to 200, or preferably from 0 to 100, or more preferably from 0 to 50, or even more preferably from 0 to 20,
s=0 to 200, or preferably from 0 to 100, or more preferably from 0 to 50, or even more preferably from 0 to 20,
and q+r+s = 1 to 600, or preferably from 1 to 100, or more preferably from 1 to 50, or even more preferably from 1 to 40.

For polyorganosiloxane structural units with the general formula S: 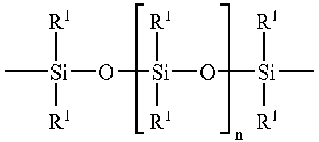 R¹=C₁-C₂₂-alkyl, C₁-C₂₂-fluoralkyl or aryl; n= from 200 to 1000, or preferably from 300 to 500, K (in the group -K-S-K-) is preferably a bivalent or trivalent straight chain, cyclical or branched C₂-C₂₀ hydrocarbon residue which is optionally interrupted by-O-,-NH-, trivalent N,-NR¹-,-C(O)-,-C(S)-, and optionally substituted with-OH.

In specific embodiments, R¹ is C₁-C₁₈ alkyl, C₁-C₁₈ fluoroalkyl and aryl. Furthermore, R¹ is preferably C₁-C₁₈ alkyl, C₁-C₆ fluoroalkyl and aryl. Furthermore, R¹ is more preferably C₁-C₆ alkyl, C₁-C₆ fluoroalkyl, even more preferably C₁-C₄ fluoroalkyl, and phenyl. Most preferably, R¹ is methyl, ethyl, trifluoropropyl and phenyl.

As used herein, the term "C₁-C₂₂ alkyl" means that the aliphatic hydrocarbon groups possess from 1 to 22 carbon atoms which can be straight chain or branched. Methyl, ethyl, propyl, n-butyl, pentyl, hexyl, heptyl, nonyl, decyl, undecyl, isopropyl, neopentyl and 1,2,3-trimethyl hexyl moieties serve as examples.

Further as used herein, the term "C₁-C₂₂ fluoroalkyl" means aliphatic hydrocarbon compounds with 1 to 22 carbon atoms which can be straight chain or branched and are substituted with at least one fluorine atom. Monofluormethyl, monofluoroethyl, 1,1,1-trifluorethyl, perfluoroethyl, 1,1,1-trifluoropropyl, 1,2,2-trifluorobutyl are suitable examples.

Moreover, the term "aryl" means unsubstituted or phenyl substituted once or several times with OH, F, Cl, CF₃, C₁-C₆ alkyl, C₁-C₆ alkoxy, C₃-C₇ cycloalkyl, C₂-C₆ alkenyl or phenyl. Aryl may also mean naphthyl.

For the embodiments of the polyorganosiloxanes, the positive charges resulting from the ammonium group(s), are neutralized with inorganic anions such as chloride, bromide, hydrogen sulfate, sulfate, or organic anions, like carboxylates deriving from C₁-C₃₀ carboxylic acids, for example acetate, propionate, octanoate, especially from C₁₀-C₁₈ carboxylic acids, for example decanoate, dodecanoate, tetradecanoate, hexadecanoate, octadecanoate and oleate, alkylpolyethercarboxylate, alkylsulphonate, arylsulphonate, alkylarylsulphonate, alkylsulphate, alkylpolyethersulphate, phosphates derived from phosphoric acid mono alkyl/aryl ester and phosphoric acid dialkyl/aryl ester. The properties of the polyorganosiloxane compounds can be, inter alia, modified based upon the selection of acids used.

The quaternary ammonium groups are usually generated by reacting the di-tertiary amines with an alkylating agents, selected from in particular di-epoxides (sometimes referred to also as bis-epoxides) in the presence of mono carboxylic acids and difunctional dihalogen alkyl compounds.

In a preferred embodiment the polyorganosiloxane compounds are of the general formulas (Ia) and (Ib):

M-Y-[-(N⁺R₂-T-N⁺R₂)-Y-]ₘ-[-(NR²-A-E-A'-NR²)-Y-]ₖ-M (Ia)

M-Y-[-(N⁺R₂-T-N⁺R₂)-Y-]ₘ L-(N⁺R²₂-A-E-A'-N⁺R²₂)-Y-]ₖ-M (Ib)

wherein each group is as defined above; however, the repeating units are in a statistical arrangement (i.e., not a block-wise arrangement).

In a further preferred embodiment the polyorganosiloxane compounds may be also of the general formulas (IIa) or (IIb):

M-Y-[-N⁺R₂-Y-]ₘ-[-(NR²-A-E-A'-NR²)-Y-]ₖ-M (IIa)

M-Y-[-N⁺R₂-Y-]ₘ-[-(N⁺R²₂-A-E-A'-N⁺R²₂)-Y-]ₖ-M (IIb)

wherein each group is as defined above. Also in such formula the repeating units are usually in a statistical arrangement (i.e not a block-wise arrangement).
wherein, as defined above, M is

-OC(O)-Z,

-OS(O)₂-Z

-OS(O₂)O-Z

-OP(O)(O-Z)OH

-OP(O)(O-Z)₂

Z is a straight chain, cyclic or branched saturated or unsaturated C₁-C₂₀, or preferably C₂ to C₁₈, or even more preferably a hydrocarbon radical, which can be interrupted by one or more -O-, or -C(O)- and substituted with -OH. In a specific embodiment, M is -OC(O)-Z resulting from normal carboxylic acids in particular with more than 10 carbon atoms like for example dodecanoic acid.

In a further embodiment, the molar ratio of the polyorganosiloxane-containing repeating group -K-S-K-and the polyalkylene repeating group -A-E-A'- or -A'-E-A- is between 100:1 and 1:100, or preferably between 20:1 and 1:20, or more preferably between 10:1 and 1:10.

In the group -(N⁺R₂-T-N⁺R₂)-, R may represent a monovalent straight chain, cyclic or branched C₁-C₂₀ hydrocarbon radical, which can be interrupted by one or more -O- , - C(O)- and can be substituted by-OH, T may represent a divalent straight-chain, cyclic, or branched C₁-C₂₀ hydrocarbon radical, which can be interrupted by -O- , -C(O)- and can be substituted by hydroxyl.

The above described polyorganosiloxane compounds comprising quaternary ammonium functions and ester functions may also contain: 1) individual molecules which contain quaternary ammonium functions and no ester functions; 2) molecules which contain quaternary ammonium functions and ester functions; and 3) molecules which contain ester functions and no quaternary ammonium functions. While not limited to structure, the above described polyorganosiloxane compounds comprising quaternary ammonium functions and ester functions are to be understood as mixtures of molecules comprising a certain averaged amount and ratio of both moieties.

Various monofunctional organic acids may be utilized to yield the esters. Exemplary embodiments include C₁-C₃₀ carboxylic acids, for example C₂, C₃, C₈ acids, C₁₀-C₁₈ carboxylic acids, for example C₁₂, C₁₄, C₁₆ acids, saturated, unsaturated and hydroxyl functionalized C₁₈ acids, alkylpolyethercarboxylic acids, alkylsulphonic acids, arylsulphonic acids, alkylarylsulphonic acids, alkylsulphuric acids, alkylpolyethersulphuric acids, phosphoric acid mono alkyl/aryl esters and phosphoric acid dialkyl/aryl esters.

### SOLUBLE SALT

The composition of the present invention may further contain a soluble salt, in view of effective transformation of such cationic surfactant system, together with high melting point fatty compounds, to emulsion especially gel matrix.

The soluble salt is included at a level by weight of the composition of, preferably from about 0.05% to about 5%, more preferably from about 0.05% to about 2%, still more preferably from about 0.08% to about 1%.

It is preferred that the weight ratio of the soluble salt to the acid for the mono-alkyl amine salt cationic surfactant is from about 1:0.5 to about 1: 10, more preferably from about 1:1 to about 1:8, still more preferably from about 1:1.5 to about 1:5.

Such soluble salts useful herein are those having a solubility in water at 25°C of preferably 0.5g/100g water or more, more preferably 1g/100g water or more, still more preferably 1.5g/100g water or more, even more preferably 3g/100g water or more.

Such soluble salts useful herein include, for example, disodium EDTA, sodium chloride, sodium citrate, sodium carbonate, and mixtures thereof.

The soluble salt can be added anytime, for example, can be added to the aqueous phase prior to the formation of emulsions, or can be added to the emulsion prior to/together with/after the addition of optional ingredients. Preferably, the soluble salt is added to the aqueous phase prior to the formation of emulsions.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione.

### PRODUCT FORMS and METHOD OF USE

The compositions of the present invention can be in the form of rinse-off products or leave-on products, and can be formulated in a wide variety of product forms, including but not limited to creams, gels, emulsions, mousses and sprays. The composition of the present invention is especially suitable for hair conditioners especially rinse-off hair conditioners.

The composition of the present invention is preferably used for a method of conditioning hair, the method comprising following steps:
(i) after shampooing hair, applying to the hair an effective amount of the conditioning composition for conditioning the hair; and
(ii) then rinsing the hair.

### EXAMPLES

The following examples further describe and demonstrate embodiments within the scope of the present invention. Where applicable, ingredients are identified by chemical or CTFA name, or otherwise defined below.

**Table 1: Compositions (wt%)**

| | Components | Ex.1 | Ex.2 | Ex.3 | CEx.i | CEx.ii | CEx.iii | CEx.iv |
|---|---|---|---|---|---|---|---|---|
| 1 | Stearamidopropyldimethylamine | 1.17 | 1.17 | - | 1.17 | - | 1.17 | 1.17 |
| 2 | Behenamidopropyldimethylamine | - | - | 1.17 | - | - | - | - |
| 3 | Varisoft 432 PPG *1 | 0.55 | 0.55 | 0.55 | 0.55 | 0.62 | 0.55 | 0.55 |
| 4 | Behentrimonium methosulfate | - | - | - | - | 1.83 | - | - |
| 5 | Cetyl alcohol | 1.1 | 1.1 | 1.1 | 1.1 | 1.1 | 1.1 | 1.1 |
| 6 | Stearyl alcohol | 2.8 | 2.8 | 2.8 | 2.8 | 2.8 | 2.8 | 2.8 |
| 7 | Benzyl alcohol | 0.4 | 0.4 | 0.4 | 0.4 | 0.4 | 0.4 | 0.4 |
| 8 | 1-Glutamic acid | 0.37 | 0.37 | 0.37 | 0.37 | 0.37 | 0.37 | 0.37 |
| 9 | Disodium EDTA | 0.13 | 0.13 | 0.13 | 0.13 | 0.13 | 0.13 | 0.13 |
| 10 | Water-soluble preservatives | 0.03 | 0.03 | 0.03 | 0.03 | 0.03 | 0.03 | 0.03 |
| 11 | Deionized Water | q.s. to 100% of the composition | | | | | | |
| 12 | Silicone compound-1 *2 | 0.35 | 0.35 | 0.35 | 0.35 | 0.35 | 0.35 | 0.35 |
| 13 | Silicone compound-2 *3 | - | - | - | - | - | - | - |
| 14 | Silicone compound-3 *4 | - | - | - | - | - | - | - |
| 15 | Deposition polymer-1 *5 | 0.5 | - | 0.5 | - | 0.5 | - | - |
| 16 | Deposition polymer-2 *6 | - | 0.5 | - | - | - | - | - |
| 17 | Comparative deposition polymer *7 | - | - | - | - | - | 0.5 | - |
| 18 | Comparative deposition polymer *8 | - | - | - | - | - | - | 0.5 |
| Silicone deposition on damaged hair | | A1+ | - | - | C1 | C1+ | C1- | D1 |

**Table 2: Compositions (wt%)**

| | Components | Ex.4 | Ex.5 | CEx.v | CEx.vi |
|---|---|---|---|---|---|
| 1 | Stearamidopropyldimethylamine | 0.90 | 0.90 | 0.90 | 0.90 |
| 2 | Behenamidopropyldimethylamine | - | - | - | - |
| 3 | Varisoft 432 PPG *1 | 0.43 | 0.43 | 0.43 | 0.43 |
| 4 | Behentrimonium methosulfate | - | - | - | - |
| 5 | Cetyl alcohol | 1.43 | 1.43 | 1.43 | 1.43 |
| 6 | Stearyl alcohol | 2.58 | 2.58 | 2.58 | 2.58 |
| 7 | Benzyl alcohol | 0.40 | 0.40 | 0.40 | 0.40 |
| 8 | 1-Glutamic acid | 0.29 | 0.29 | 0.29 | 0.29 |
| 9 | Disodium EDTA | 0.13 | 0.13 | 0.13 | 0.13 |
| 10 | Water-soluble preservatives | 0.03 | 0.03 | 0.03 | 0.03 |
| 11 | Deionized Water | q.s. to 100% of the composition | | | |
| 12 | Silicone compound-1 *2 | - | - | - | - |
| 13 | Silicone compound-2 *3 | 0.5- | - | 0.5- | - |
| 14 | Silicone compound-3 *4 | - | 0.5 | - | 0.5 |
| 15 | Deposition polymer-1 *5 | 0.5 | 0.5 | - | - |
| 16 | Deposition polymer-2 *6 | - | - | - | - |
| 17 | Comparative deposition polymer *7 | - | - | - | - |
| 18 | Comparative deposition polymer *8 | - | - | - | - |
| Silicone deposition on damaged hair | | S2+ | S2+ | C2+ | C2 |

### Definitions of Components

*1 67-69% of Dicetyldimonium Chloride in q.s. to 100% Propylene Glycol and 5% water, available from Evonik Goldschmidt Corporation
*2 Silicone compound-1: Available from Momentive having the following formula:

M-Y-[-(N⁺R₂-T-N⁺R₂)-Y-]ₘ-[-(N⁺R²₂-A-E-A'-N⁺R²₂)-Y-]ₖ-M

wherein

| | |
|---|---|
| M | lauric ester |
| Y | K-S-K |
| K | CH₂-CHOH-CH₂-O-C₃H₆ |
| S | PDMS block with 368 siloxane units |
| R, R² | Methyl |
| T | C₆H₁₂ |
| A | CH₂-COO- |
| A' | CO-CH₂ |
| E | Ethylene oxide (CH₂-CH₂-O) with average degree of ethoxylation of 2 |
| Ratio of silicone blocks : alkylene oxide blocks | 1:1 |
| Total Viscosity | 4700 mPa•s |

*3 Silicone compound-2: Available from Momentive having a viscosity 10,000mPa·s, and having following formula (I):

(R₁)ₐG₃₋ₐ-Si-(-OSiG₂)ₙ-(-OSiG_{b}(R₁)_{2-b})ₘ-O-SiG₃₋ₐ(R₁)ₐ (I)

wherein G is methyl; a is an integer of 1; b is 0, 1 or 2, preferably 1; n is a number from 400 to about 600; m is an integer of 0; R₁ is a monovalent radical conforming to the general formula CqH_{2q}L, wherein q is an integer of 3 and L is -NH₂
*4 Silicone compound-3: Dimethicone having a viscosity of 10,000mPa•s.
*5 Deposition polymer-1: Copolymer of 30wt%of acrylic acid monomer and 70wt% of methoxyPEG-4methacrylate monomer, having a molecular weight of about 24,000, available from Mitsubishi Chem.
*6 Deposition polymer-2: Copolymer of 50wt%of acrylic acid monomer and 50wt% of methoxyPEG-4methacrylate monomer, having a molecular weight of about 24,000, available from Mitsubishi Chem.
*7 Comparative deposition polymer: Copolymer of AA/GLM=30/70 (AA stands for acrylic acid; GLM stands for glycerine methacrylate having the following formula: CH2=C(CH3)COO-CH2-CH(OH)-CH2OH)), having a molecular weight of about 130,000, available from Mitsubishi Chem.
*8 Comparative deposition polymer: Copolymer of AA/HEAA/SMA=30/50/20 (AAstands for acrylic acid; HEAA stands for N-(2-hydroxyethyl)acrylamide); SMA stands for stearylmethacrylate), having a molecular weight of about30,000, available from Mitsubishi Chem.

### Method of Preparation

The above hair conditioning compositions of "Ex. 1" through "Ex. 5" and "CEx. i" through "CEx. vi" were prepared by the following method:
Components 1-7 are mixed and heated to from about 66°C to about 85°C to form an oil phase. Separately, Components 8-11 are mixed and heated to from about 20°C to about 48°C to form an aqueous phase. In Becomix® direct injection rotor-stator homogenizer, the oil phase is injected and it takes 0.2 second or less for the oils phase to reach to a high shear field having an energy density of from 1.0x10⁵ J/m³ to 1.0x10⁷ J/m³ where the aqueous phase is already present. A gel matrix is formed. Components 12-18 are added to the gel matrix with agitation. Then the composition is cooled down to room temperature.

### Properties and Conditioning benefits

For some of the above compositions, properties and conditioning benefits are evaluated by the following methods. Results of the evaluation are also shown above.

The embodiments disclosed and represented by "Ex. 1" through "Ex. 5" are hair conditioning compositions of the present invention which are particularly useful for rinse-off use. Such embodiments have many advantages. For example, they provide improved deposition of silicone compound on damaged hair.

Such advantages can be understood by the comparison between the examples of the present invention and comparative examples "CEx. i" through "CEx. vi". For example, significantly improved silicone deposition was observed in "Ex. 1" of the present invention, compared to comparative examples "CEx. i" containing no deposition polymer, "CEx. ii" using a different cationic surfactant, and "CEx.iii" and "CEx. iv" both using different deposition polymers. Significantly improved silicone depositions were also observed in different types of silicones, for example, those used in "Ex. 4" and "Ex. 5" of the present invention, compared to comparative examples "CEx. v" and "CEx. vi" both containing no deposition polymer.

The amount of the silicone deposition can be measured and evaluated by a method consisting of: (i) a preparation of hair switch; and (ii) silicone deposition measurement, and (iii) evaluation.

### (i) Preparation of hair switch

For the silicone deposition measurement, 2 gram hair switches are used. The hair switches are prepared by following steps:
(1) The hair switches are bleached and combed in the same way. Then, applying 0.2g of non-conditioning shampoo per one hair switch, lathering, rinsing and drying the hair switches;
(2) Applying a non-conditioning shampoo at a level of 0.2cc per one hair switch and lathering the hair switch; and rinsing the hair switch;
(3) Applying a non-conditioning shampoo again at a level of 0.2cc per one hair switch and lathering the hair switch; and rinsing the hair switch; and
(4) Applying a conditioner at a level of 0.2cc per one hair switch and treating the hair switch; and rinsing the hair switch; and
(5) Then drying the hair switch.

The hair switch is ready for the measurement of its silicone deposition amount, and evaluation.

### (ii) Silicone deposition measurement

The deposited silicone on the hair switch is extracted in an appropriate solvent. The extracts are then introduced into an atomic absorption/emission detector instrument and measured at the appropriate wavelength. The absorbance/emission value returned by the instrument is then converted to actual concentration (ppm) of silicone compound deposited on the hair through an external calibration curve obtained with known weights of a well characterized standard of the silicone compound under study.

### (iii) Evaluation

### In Table 1

| | |
|---|---|
| A1+: | Above 100% (Excluding 100%) to 150% increased deposition, compared to Control. |
| A1: | Above 60% (Excluding 60%) to 100% increased deposition, compared to C1. |
| B1: | Above 25% (Excluding 25%) to 60% increased deposition, compared to C1. |
| C1+: | Up to 25% increased deposition, compared to C1 |
| C1: | Control |
| C1-: | Up to 25% decreased deposition, compared to C1. |
| D1: | Above 25% (Excluding 25%) to 60% decreased deposition, compared to C1. |

### In Table 2

| | |
|---|---|
| S2+: | Above 200% (Excluding 200%) to 300% increased deposition, compared to C2. |
| S2: | Above 150% (Excluding 150%) to 200% increased deposition, compared to C2. |
| A2+: | Above 100% (Excluding 100%) to 150% increased deposition, compared to C2. |
| A2: | Above 60% (Excluding 60%) to 100% increased deposition, compared to C2. |
| B2: | Above 25% (Excluding 25%) to 60% increased deposition, compared to C2. |
| C2+: | Up to 25% increased deposition, compared to C2. |
| C2: | Another Control |
| C2-: | Up to 25% decreased deposition, compared to C2. |
| D2: | Above 25% (Excluding 25%) to 60% decreased deposition, compared to C2. |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair conditioning composition comprising by weight:
(a) from 0.1% to 8% of a primary, secondary or tertiary alkyl amine cationic surfactant having one long alkyl or alkenyl group of from about 12 to about 30 carbon atoms;
(b) from 0.05% to 6% of a di-alkyl quaternized ammonium salt cationic surfactant said di-alkyl groups being two long alkyl groups of from about 12 to about 30 carbon atoms;
(c) from 1% to 15% of a high melting point fatty compound;
(d) from 0.05% to 6% of a deposition polymer which is a copolymer comprising: a vinyl monomer (A) with a carboxyl group in the structure; and a vinyl monomer (B) expressed by the following formula (1):
CH₂=C(R¹)-CO-X-(Q-O)ᵣ-_{R}² (1)
wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and wherein the vinyl monomer (A) is contained at a level of from about 10 mass% to about 50 mass%, and the vinyl monomer (B) is contained at level of from about 50 mass% to about 90 mass%; and
(e) from 0.05% to 15% of a silicone compound; and
(f) an aqueous carrier.

2. The composition of Claim 1 wherein, in the formula (1), r represents from 3 to about 12.

3. The composition of Claim 1 wherein, in the formula (1), X represents an oxygen atom.

4. The composition of Claim 1 wherein the vinyl monomer (A) is expressed by the following formula (2) or the following formula (3):
CH₂=C(R³)-CO-(O-(CH₂)ₘ-CO)ₙ-OH (2)
wherein R³ represents a hydrogen atom or a methyl group, m represents an integer of 1 through 4, and n represents an integer of 0 through 4;
CH₂=C(R⁴)-COO-(CH₂)ₚ-OOC-(CH₂)_{q}-COOH (3)
wherein R⁴ represents a hydrogen atom or a methyl group, p and q independently represent an integer of 2 through 6.

5. The composition of Claim 1 wherein the deposition polymer has a weighted average molecular weight of from 3,000 to 2,000,000.

6. The composition of Claim 1 wherein the deposition polymer is anionic.

7. The hair conditioning composition of Claim 1 wherein the amine is a tertiary amidoamine having an alkyl group of from about 16 to about 22 carbon atoms.

8. The hair conditioning composition of Claim 1 wherein cationic component a) is used in combination with an acid selected from the group consisting of ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof, at a level such that the mole ratio of the amine to the acid is from about 1 : 0.3 to about 1 : 2.

9. The composition of Claim 1 wherein the silicone compound is selected from polyalkyl siloxanes, polyaryl siloxanes, polyalkylaryl siloxanes, polyether siloxane copolymers, amino substituted silicones, quaternized silicones including quaternized silicones comprising terminal ester groups, having a viscosity of lower than 100,000 mPa·s and a D block length of greater than 200 D units, and mixtures thereof.

## Patentansprüche

1. Haarkonditionierungszusammensetzung, umfassend, in Gewichtsprozent:
(a) von 0,1 % bis 8% kationisches Tensid in Form von primärem, sekundärem oder tertiärem Alkylamin mit einer langen Alkyl- oder Alkenylgruppe mit etwa 12 bis etwa 30 Kohlenstoffatomen;
(b) von 0,05 % bis 6 % kationisches Tensid in Form eines quaternisierten Dialkylammoniumsalzes, wobei die Dialkylgruppen zwei lange Alkylgruppen mit etwa 12 bis etwa 30 Kohlenstoffatomen sind;
(c) von 1 % bis 15 % eine Fettverbindung mit hohem Schmelzpunkt;
(d) von 0,05 % bis 6 % ein Anlagerungspolymer, bei dem es sich um ein Copolymer handelt, das Folgendes umfasst: ein Vinylmonomer (A) mit einer Carboxylgruppe in der Struktur; und ein Vinylmonomer (B), ausgedrückt durch die folgende Formel (1):
CH₂=C(R¹)-CO-X-(Q-O)ᵣ-R² (1)
wobei: R¹ für ein Wasserstoffatom oder eine Methylgruppe steht; R² für ein Wasserstoffatom oder eine Alkylgruppe mit 1 bis 5 Kohlenstoffatomen steht, die eine Substitutionsgruppe aufweisen können; Q für eine Alkylengruppe mit 2 bis 4 Kohlenstoffatomen steht, die ebenfalls eine Substitutionsgruppe aufweisen können; r für eine ganze Zahl von 2 bis 15 steht; und X für ein Sauerstoffatom oder eine NH-Gruppe steht; und, in der folgenden Struktur - (Q - O)ᵣ - R², die Anzahl der in einer geraden Kette gebundenen Atome 70 oder weniger beträgt; und wobei das Vinylmonomer (A) in einer Konzentration von etwa 10 Massen-% bis etwa 50 Massen-% enthalten ist, und das Vinylmonomer (B) in einer Konzentration von etwa 50 Massen-% bis etwa 90 Massen-% enthalten ist; und
(e) von 0,05 % bis 15 % eine Silikonverbindung; und
(f) einen wässrigen Träger.

2. Zusammensetzung nach Anspruch 1, wobei in der Formel (1) r für 3 bis etwa 12 steht.

3. Zusammensetzung nach Anspruch 1, wobei in der Formel (1) X für ein Sauerstoffatom steht.

4. Zusammensetzung nach Anspruch 1, wobei das Vinylmonomer (A) durch die folgende Formel (2) oder die folgende Formel (3) ausgedrückt wird:
CH₂=C(R³)-CO-(O-(CH₂)ₘ-CO)ₙ-OH (2)
wobei R³ für ein Wasserstoffatom oder eine Methylgruppe steht, m für eine ganze Zahl von 1 bis 4 steht, und n für eine ganze Zahl von 0 bis 4 steht;
CH₂=C(R⁴)-COO-(CH₂)ₚ-OOC-(CH₂)_{q}-COOH (3)
wobei R⁴ für ein Wasserstoffatom oder eine Methylgruppe steht, p und q unabhängig für eine ganze Zahl von 2 bis 6 stehen.

5. Zusammensetzung nach Anspruch 1, wobei das Anlagerungspolymer ein durchschnittliches Molekulargewicht (Gewichtsmittel) von 3.000 bis 2.000.000 aufweist.

6. Zusammensetzung nach Anspruch 1, wobei das Anlagerungspolymer anionisch ist.

7. Haarkonditionierungszusammensetzung nach Anspruch 1, wobei das Amin ein tertiäres Amidoamin mit einer Alkylgruppe mit etwa 16 bis etwa 22 Kohlenstoffatomen ist.

8. Haarkonditionierungszusammensetzung nach Anspruch 1, wobei die kationische Komponente a)
in Kombination mit einer Säure verwendet wird, die ausgewählt ist aus der Gruppe bestehend aus ℓ-Glutaminsäure, Milchsäure, Chlorwasserstoffsäure, Äpfelsäure, Bernsteinsäure, Essigsäure, Fumarsäure, Weinsäure, Zitronensäure, ℓ-Glutaminhydrochlorid, Maleinsäure und deren Mischungen, in einem solchen Anteil, dass das Molverhältnis des Amins zur Säure etwa folgendes ist: 1 : 0,3 bis etwa 1 : 2.

9. Zusammensetzung nach Anspruch 1, wobei die Silikonverbindung ausgewählt ist aus Polyalkylsiloxanen, Polyarylsiloxanen, Polyalkylarylsiloxanen, Polyether-Siloxan-Copolymeren, aminosubstituierten Silikonen, quaternisierten Silikonen einschließlich quaternisierter Silikone, die endständige Estergruppen aufweisen, mit einer Viskosität von unter 100.000 mPa.s und einer D-Blocklänge von über 200 D-Einheiten, und deren Mischungen.

## Revendications

1. Composition de conditionnement des cheveux comprenant en poids :
(a) de 0,1 % à 8% d'un agent tensioactif cationique alkylamine primaire, secondaire ou tertiaire comportant un groupe alkyle ou alcényle long allant d'environ 12 à environ 30 atomes de carbone ;
(b) de 0,05 % à 6 % d'un agent tensioactif cationique de sel de di-alkyl ammonium quaternisé lesdits groupes di-alkyle étant deux groupes alkyle longs allant d'environ 12 à environ 30 atomes de carbone ;
(c) de 1 % à 15 % d'un composé gras à point de fusion élevé ;
(d) de 0,05 % à 6 % d'un polymère de dépôt qui est un copolymère comprenant : un monomère vinylique (A) avec un groupe carboxyle dans la structure ; et un monomère vinylique (B) exprimé par la formule (1) suivante :
CH₂=C(R¹)-CO-X-(Q-O)ᵣ-R² (1)
dans laquelle : R¹ représente un atome d'hydrogène ou un groupe méthyle ; R² représente un atome d'hydrogène ou un groupe alkyle avec de 1 à 5 atomes de carbone, qui peuvent avoir un groupe de substitution ; Q représente un groupe alkylène avec de 2 à 4 atomes de carbone qui peuvent également avoir un groupe de substitution ; r représente un nombre entier allant de 2 à 15 ; et X représente un atome d'oxygène ou un groupe NH ; et, dans la structure suivante - (Q - O)ᵣ - R², le nombre d'atomes liés dans une chaîne linéaire est 70 ou moins ; et dans laquelle le monomère vinylique (A) est contenu à un taux allant d'environ 10 % en masse à environ 50 % en masse, et le monomère vinylique (B) est contenu à un taux allant d'environ 50 % en masse à environ 90 % en masse ; et
(e) de 0,05 % à 15 % d'un composé de silicone ; et
(f) un véhicule aqueux.

2. Composition selon la revendication 1, dans laquelle, dans la formule (1), r représente de 3 à environ 12.

3. Composition selon la revendication 1, dans laquelle, dans la formule (1), X représente un atome d'oxygène.

4. Composition selon la revendication 1, dans laquelle le monomère vinylique (A) est exprimé par la formule (2) suivante ou la formule (3) suivante :
CH₂=C(R³)-CO-(O-(CH₂)ₘ-CO)ₙ-OH (2)
dans laquelle R³ représente un atome d'hydrogène ou un groupe méthyle, m représente un nombre entier de 1 à 4, et n représente un nombre entier de 0 à 4 ;
CH₂=C(R⁴)-COO-(CH₂)ₚ-OOC-(CH₂)_{q}-COOH (3)
dans laquelle R⁴ représente un atome d'hydrogène ou un groupe méthyle, p et q représentent indépendamment un nombre entier de 2 à 6.

5. Composition selon la revendication 1, dans laquelle le polymère de dépôt a une masse moléculaire moyenne en poids allant de 3000 à 2 000 000.

6. Composition selon la revendication 1, dans laquelle le polymère de dépôt est anionique.

7. Composition de conditionnement des cheveux selon la revendication 1, dans laquelle l'amine est une amido-amine tertiaire possédant un groupe alkyle allant d'environ 16 à environ 22 atomes de carbone.

8. Composition de conditionnement des cheveux selon la revendication 1, dans laquelle le composant cationique a)
est utilisé en combinaison avec un acide choisi dans le groupe constitué d'acide ℓ-glutamique, acide lactique, acide chlorhydrique, acide malique, acide succinique, acide acétique, acide fumarique, acide tartrique, acide citrique, chlorhydrate ℓ-glutamique, acide maléique, et leurs mélanges, à un taux tel que le rapport molaire de l'amine à l'acide va d'environ 1: 0,3 à environ 1: 2.

9. Composition selon la revendication 1, dans laquelle le composé de silicone est choisi parmi des polyalkyl-siloxanes, des polyaryl-siloxanes, des polyalkylaryl-siloxanes, des copolymères de polyéther et de siloxane, des silicones à substitution amino, des silicones quaternisées incluant des silicones quaternisées comprenant des groupes ester terminaux, ayant une viscosité inférieure à 100 000 mPa.s et une longueur de motif D supérieure à 200 motifs D, et leurs mélanges.
